# EUROPEAN PATENT SPECIFICATION

(11) **EP 0 372 071 B1**
(45) Date of publication and mention of the grant of the patent: **16.08.1995**
(21) Application number: 89907912.3
(22) Date of filing: 08.06.1989
(51) Int. Cl.: C12N 5/12, C07K 16/18, A61K 39/395, A61K 45/05

(54) **MONOCLONAL ANTIBODIES AGAINST LENS EPITHELIAL CELLS AND METHODS FOR PREVENTING PROLIFERATION OF REMNANT LENS EPITHELIAL CELLS AFTER EXTRACAPSULAR EXTRACTION**
MONOKLONALE ANTIKÖRPER GEGEN LINSENEPITHELZELLEN UND METHODEN ZUR VERHINDERUNG DES VERMEHRUNG ÜBRIGGEBLIEBENER LINSENEPITHELZELLEN NACH EXTRAKAPSULÄRES EXTRAKTION
ANTICORPS MONOCLONAUX DRESSES CONTRE DES CELLULES EPITHELIALES DU CRISTALLIN ET PROCEDES EMPECHANT LA PROLIFERATION DE CELLULES EPITHELIALES RESTANTES DU CRISTALLIN APRES EXTRACTION EXTRACAPSULAIRE

(30) Priority: 08.06.1988 US 204168; 14.06.1988 US 206610
(43) Date of publication of application: 13.06.1990
(73) Proprietor: BAYLOR COLLEGE OF MEDICINE, Houston, TX 77030 (US)
(72) Inventor: EMERY, Jared, M., Houston, TX 77030 (US); LAM, Dominic, M.-K., The Woodlands, TX 77380 (US); KELLEHER, Peter, J., The Woodlands, TX 77381 (US)
(74) Representative: Goldin, Douglas Michael
(86) International application number: US8902521
(87) International publication number: WO8912093

(56) References cited:
- Investigative Ophthalmology & Visual Science, vol. 27, Issued April 1986, B. Hsi et al. see page 620
- Journal of Experimental Medicine, vol. 166, issued July 1987, M.J. Glennie et al. see paragraph bridging pp. 44-45
- STN File CA: Chemical Abstracts, Blakey, D. et al. "Immunoxins", see abstract no. CA105(7):53985t, BioEssays, 1986, 4(6), 292-7

## Description

### INTRODUCTION

### Technical Field

The field concerns methods and compositions for inhibiting proliferation of remnant lens epithelial cells.

### Background

Extracapsular cataract extraction is a desirable method for removing cataracts. With this technique there is a lower incidence of post-operative complications such as cystoid macular edema and retinal detachment. The availability of an improved extracapsular extraction technique such as phacoemulsification and the requirement for an intact posterior lens capsule for implantation of a wide variety of intraocular lenses has further supported use of extracapsular cataract extraction for removing cataracts.

Extracapsular lens extraction is accompanied by a significant incidence of posterior lens capsule opacification, which may require additional surgical procedures such as posterior capsulotomy or repolishing of the posterior lens capsule in order to obtain good vision. The pathogenesis of posterior lens capsule opacification after extracapsular cataract extraction is due to proliferation of remnant lens epithelial cells on the posterior lens capsule to form abortive lens "fibers" and "bladder" cells ("Elschnig's pearls").

To inhibit secondary cataract formation, a variety of techniques have been used. Roy et al., Contact and Intraocular Lens Medical Journal (1979) 5:175-178 reported the use of vincristine and vinblastine as a means of inhibiting lens cell proliferation. Radiation treatment has also been tried, and reported to be promising. Instillation of methotrexate and retinoic acid into the anterior chamber of the eye has also been tried to prevent posterior lens capsule opacification. However these treatment regimens lack specificity; they do not distinguish between lens epithelial cells and other cell types present in the anterior chamber and as a result of these treatments other (desirable) cells may be damaged. It is therefore of interest to develop methods and compositions which would provide for selective destruction of remnant lens epithelial cells as a means of preventing formation of secondary cataracts and posterior lens capsule opacification.

### Relevant Literature

Production of monoclonal antibodies has been described. See, for example, Monoclonal Antibodies, eds. Roger H. Kennett, Thomas J. McKearn, Kathleen B. Bechtol, Plenum Press, New York, 1980; Nature (1975) 256:495-497; U.S. Patent Nos. 4,271,145; 4,196,265; 4,172,124; 4,195,125; 4,262,090; and 4,294,927.

### SUMMARY OF THE INVENTION

Methods and compositions are provided for inhibiting posterior lens capsule opacification after extracapsular cataract extraction. The methods involve using cytotoxic agents at least substantially specific for epithelial cells and introducing them into the anterior chamber of the eye concurrently with or subsequent to the extracapsular cataract extraction. Of particular interest is introducing non-cytotoxic agents into the anterior chamber prior to the introduction of the cytotoxic agents, where the non-cytotoxic and cytotoxic agents have substantially the same binding affinity for epithelial cells. The method is effective in preventing opacification of the posterior lens capsule.

### DESCRIPTION OF THE SPECIFIC EMBODIMENTS

Methods and compositions are provided for inhibiting proliferation of remnant lens epithelial cells after extracapsular extraction. The method comprises introducing into the anterior chamber of the eye, at the time of original cataract removal or subsequently, cytotoxic agents specific for lens epithelial cells which bind to any remnant lens epithelial cells. The cytotoxic agents are at least substantially specific for human lens epithelial cells and have low or no cross-reactivity with other cells found in the anterior chamber such as fibroblasts, melanocytes, corneal endothelial cells, etc., desirably also other epithelial cells, e.g., corneal epithelial cells. Preferably, prior to introduction of the cytotoxic agent and prior to the extracapsular cataract extraction, a non-cytotoxic agent is introduced into the anterior chamber, where the non-cytotoxic agent is cross-reactive with or has substantially the same binding specificity as the cytotoxic agent, so as to bind to any cells in the anterior chamber which are in contact with the anterior chamber having homologous determinant or antigenic sites.

The cytotoxic agent may comprise a combination of a monoclonal antibody at least substantially specific for lens epithelial cells and capable of fixing complement, and complement. In use, the antibody is introduced first into the anterior chamber of the eye. Following a sufficient time for the antibody to bind to any remnant lens epithelial cells, complement is introduced into the anterior chamber, effecting selective lysis of the lens epithelial cells. The monoclonal antibodies may be derived either from a single hybridoma cell line or may be a mixture or "cocktail" of two or more monoclonal antibodies derived from different hybridoma cell lines, where the antibodies would bind to different antigenic moieties on lens epithelial cells. For use with complement, the monoclonal antibodies may be any mammalian species, including murine, rabbit, human or the like, or combinations thereof, such as chimeric antibodies, having for example a human CH₂ domain or the Fc portion of the molecule, and a mouse or other mammalian source variable region. The antibodies may be any class or subclass which will activate complement, such as IgM, IgG, 2a, 2b, or 3 or the human equivalent thereof (for a description of complement-fixing classes and subclasses, see Monoclonal Antibodies: Principles and Practice, 2nd ed., Goding, Academic Press, New York, pg. 14, 1986, which publication is incorporated herein by reference).

Alternatively, the cytotoxic agent may comprise a conjugate of a monoclonal antibody capable of binding substantially specifically to lens epithelial cells, and a toxic agent. The toxic agent may be derived from microorganism or a plant source. Of particular interest are the toxic subunits of naturally occurring toxins, such as ricin, abrin, diphtheria toxin, etc. See for example Oeltmann and Heath, J. Biol. Chem. (1979) 254:1022-1027; Yule and Neville Jr., Proc. Natl. Acad. Sci. USA (1980) 77:5483-5486; Gilliland et al., Proc. Natl. Acad. Sci. USA (1978) 75:5319-5323; U.S. Patent No. 4,379,145; GB2034324 and Masuho et al., Biochem. Biophys. Res. Comun. (1979) 90:320-326; and Blythman, Nature (1981) 290:145, the relevant disclosures of which are incorporated herein by reference.

Illustrative toxin A-chains or similarly effective moieties include diphtheria toxin A-chains, enzymically active proteolytic fragments from Pseudo- monas aeruginosa exotoxin-A, ricin toxin A-chain, abrin A-chain, modeccin A-chain, and proteins found in various plants having similar activity, such as the plants e.g., Gelonium multiflorum,Phytolacca americana, Croton tiglium, Jatropha curcas, Momordic charantia, and wheat germ. Of particular interest is the toxin saporin from Saponaria officinalis (Thorpe et al., J. Natl. Cancer Inst. (1985) 75:151) and the plant toxin trichosanthin, and related compounds. Also, mutant species of the toxins of the species may be used, such as CRM45 (Boquet et al., Proc. Natl. Acad. Sci. USA (1976) 73:4449-4453). The monoclonal antibody may be the entire antibody molecule or a monoclonal antibody fragment, such as Fab, F(ab')₂, Fv, a recombinant variable region, a T-cell receptor, or the like. The monoclonal antibodies and receptors may be any mammalian species, including murine, rabbit, human or the like, or combinations thereof, such as chimeric antibodies, having a human constant region and a mouse or other mammalian source variable region. The antibodies may be any class or subclass, such as IgA, IgD, IgG, IgM, and may include IgGl, 2a, 2b, or 3 or the human equivalents thereof.

The methods for preparing monoclonal antibodies are well established. They may be prepared by any methods known to those skilled in the art. Various lens epithelial cells may be used as the immunogen, particularly human lens epithelial cells, although other species may find use, e.g., primates. Whole cells are preferred, however, homogenates, membrane fragments or the like, can be used. The source of the cells includes cells growing in tissue culture, or tissues removed during cataract surgery. Thus, human lens epithelial cells from tissues removed during cataract surgery or within a short time after death, preferably within approximately 30 minutes after death may be used directly, or the cells may be maintained by placing the surgical specimens in tissue culture using well established techniques.

In accordance with the subject invention, a mammal, conveniently a mouse or other small mammal, is hyperimmunized with the immunogen. Methods of immunization are well known and are amply described in the literature. The immunogenic material, generally about 10⁵ to about 10⁶ cells or cell equivalents, is injected with or without adjuvant into the mammal, or by repeated injections over relatively short periods of time. To ensure the hyperimmunization of the animal, 2-6 subsequent booster injections are administered. The animals are then killed, usually within 1-5 days after the last injection. The spleen is removed, and the spleen cells immortalized, usually by fusion with an appropriate myeloma cell line.

The method of cell fusion is not a critical portion of this invention and various techniques may be employed. Generally a nonionic detergent, for example polyethylene glycol (PEG), is used as the fusogen. The spleen cells and the myeloma cells are combined in the presence of the nonionic detergent, conveniently PEG 1540 and other additives, for example, serum-free Dulbecco's Modified Eagle's Medium (SF-DMEM), for approximately 5 minutes. The excess nonionic detergent is then rapidly removed by washing the cells.

The cells are promptly dispensed into small culture wells at a relatively low density, ranging from about 1x10⁵/well to about 5x10⁵/well in appropriate medium, commonly a selective medium comprising hypoxanthine, aminopterin and thymidine (HAT) medium.

After a sufficient period, usually one to two weeks, colonies of hybrids are observed. The colonies are then screened for antibodies which bind specifically to lens epithelial cells. If complement-fixing antibodies are required, the colonies additionally are screened to determine whether the antibodies are capable of fixing complement. Once colonies producing the desired antibodies have been identified, the colonies may be perpetuated to provide for a continued source of the desired antibodies.

To obtain hybridoma cell lines secreting monoclonal antibodies directed to a single antigenic determinant associated with lens epithelial cells, the hybridoma cells may be cloned using for example, limiting dilution. The stage at which the cells may be cloned is not critical to the invention, and may be before identification of colonies secreting antibodies of interest, or later. However, to avoid overgrowth of antibody-producing cells with non-antibody producing cells, the colonies are preferably cloned as soon after fusion as practicable.

For large scale production of antibodies, the hybridomas may introduced into the peritoneal cavity of a mammal and grown as an ascites tumor. Antibodies may then be isolated from the ascites fluid. Alternate methods for large scale production of monoclonal antibodies include inducing subcutaneous tumors using the method described above and collecting the blood from the animal. The hybridoma cells can also be grown on a large scale in tissue culture. Where the cells secrete the antibodies into the growth medium, the conditioned growth medium containing the antibodies can be collected for antibody isolation. Where the hybridoma cells do not secrete the antibodies, the cells may be collected, lysed using conventional means, and antibody purified from the cell lysate. Methods of purifying monoclonal antibodies are well known to those skilled in the art.

The complement is a standard complement. By complement is intended normal serum of man or other vertebrates which comprises about 9 major proteins which react with antigen-antibody complexes to cause damage to cell membranes, including lysis. Complement is usually supplied as serum, for example, rabbit serum. A typical complement and its preparation useful in the present invention is described in Monoclonal Antibodies (1980), Plenum Press New York, Eds. Kennett et al., pp. 391-392, which publication is incorporated herein by reference. Alternatively, it is possible to use patient complement activity which may be obtained in sufficient concentration from aqueous humor during surgery.

For preparing cytotoxic agents comprising a conjugate of a toxic agent and moiety providing for binding to the epithelial cells, the toxic agent and antibody are usually linked with a bond which is cleavable cytoplasmically. Convenient linkages include disulfide, particularly where the toxic agent has an intrinsic sulfur, or other links, such as peptide links, urea links, thioethers, imines, amides, imides, amidines, etc. Functional groups which may find employment include carboxylic acid groups, amino groups, imines, aldehydes, isocyanates, mercaptans, olefins, or the like. In addition, more complex linking groups can be employed, where a group may be bound to one of the moieties in the conjugate to provide for convenient linkage to an intrinsic group of the other moiety. For example, the N-hydroxysuccinimide ester of m-maleimidoylbenzoic acid may be employed to prepare an amide of the toxin, which may then be linked through an available sulfur atom on the monoclonal antibody to provide a thioether.

Exemplary cytotoxic agents may have the following formula:

(ASₙ)-(S-X-R)ₘ

wherein:
ASₙ indicates the toxic agent having one or more sulfur groups as part of the agent; n is 1 to the number of sulfur groups present in the toxic agent which are present as available mercaptide groups, generally being up to about 4; R is a monoclonal antibody receptor or derivative thereof; and m is 1 up to n, usually being from 1 to 2; and X is a linking group and may be a bond or a group of from about 1 to 20, usually 1 to 12 atoms other than hydrogen, which include carbon, nitrogen, oxygen and sulfur. Sulfur will normally be bonded to carbon, particularly aliphatically saturated. X may be aliphatic, alicyclic, aromatic, heterocyclic or combinations thereof, generally having from 0 to 6, more usually from about 0 to 4, preferably about 1 to 4 heteroatoms, wherein oxygen and sulfur are present as oxo or non-oxo-carbonyl or the thio analogs thereof, or ether (including thioether), and nitrogen is present as amino or amido. For the most part the heteroatoms will be bonded solely to carbon.

Illustrative groups linking the disulfide include aminoethylene 3-propanyl methylene carbonyl, α-succinimidyl, 3-propylenethiocarbonyl. The groups which may be used are for the most part conventional groups providing for a disulfide linkage. The disulfide compound is one which is capable of reacting with the cell-specific ligand, whereby a mercaptide group may be displaced from the disulfide, resulting in a new disulfide linkage between the toxic agent and the ligand.

For the most part, the linkages will be aliphatic of from about 1 to 6 carbon atoms, providing for an amide bond to the receptor, although this is primarily a matter of convenience, not necessary to the operability of the subject compositions.

Other toxic agents may also be used, such as bismuth non-diffusively linked to the monoclonal antibodies or receptors as described by Waldman, J. Amer. Med. Assoc.

Alternatively, liposomes may be linked to the monoclonal antibodies or receptors, where the liposomes contain various cytotoxic agents, such as methotrexate, 5-fluorouracil, any of the above toxins, or the like. See, for example, Szoka and Papahadjopoulos, Proc. Natl. Acad. Sci. USA (1978) 75:4194-4198 and Szoka et al., Biochem. et Biophys. Acta. (1980) 601:559-571 for the preparation of liposomes, which disclosures are incorporated herein by reference. Linking of antibodies to the liposome has been amply described in the literature, see for example Heath et al., Proc. Natl. Acad. Sci. USA (1983) 80:1377-1381 and Leserman et al., Nature (1981) 293:226-228, whose disclosures are incorporated herein by reference.

Other cytotoxic agents conjugated to the binding moiety may also be employed in conjunction with the subject process of this invention. The conjugate will be sufficient to provide the cytotoxic effect without the addition of ancillary agents.

In using the subject invention, the cytotoxic agent will be introduced into the anterior chamber of the eye following lens removal. It is preferably introduced following introduction of a non-cytotoxic agent capable of specifically binding to sites cross-reactive with the cytotoxic agent. The preferred method in carrying out the subject invention is as follows. Non-cytotoxic agent, 10-100 »g in 10-20 »l, is injected intracamerally through the limbus. For the most part, this agent will be a monoclonal antibody or a specific binding fragment thereof. Generally the solution will be a physiologically acceptable solution, which may be saline, phosphate buffered saline or the like. Other methods of introduction can include for example instillation of the non-cytotoxic agent following incision of the cornea and instillation of about 25-200, preferably about 50-150, more preferably about 100 »l of a non-cytotoxic agent capable of specifically binding to a site cross-reactive with the cytotoxic agent. The antibodies or fragments thereof will bind to all sites which may be cross-reactive with the cytotoxic agent and will also bind non-specifically to "hot spots" which may be present within the anterior chamber of the eye. In this way, the cells will be protected from the cytotoxic agent.

The use of the cross-reactive binding agent will be of particular importance where the cytotoxic agent has cross-reactivity, either specific or non-specific, with cells other than lens epithelial cells. By use of the prior instillation of the cross-reactive non-cytotoxic binding moiety, cytotoxic agents may be prepared which cross-react to varying degrees with cells, particularly epithelial cells, other than lens epithelial cells.

After allowing for antibody binding (10-15 min) a corneal incision is made (see for example Extracapsular Cataract Surgery, Emery & McIntyre, Mosby Company, St. Louis, 1983) and the anterior chamber flushed with a physiologically acceptable solution such as a balanced salt solution to remove any unbound non-cytotoxic agent. Extracapsular surgery and lens removal is performed according to standard methods (Emery & McIntyre, supra). After the corneal incision is closed, at least a second injection is made into the lens capsule area to introduce the cytotoxic composition.

Where the material for the second injection comprises a conjugate comprising a toxic moiety and the monoclonal antibody, generally the injection will comprise 10-20 »l containing an amount of the cytotoxic composition sufficient to substantially completely or completely kill all of the lens epithelial cells, usually about 1-10 »g.

Generally, the cytotoxic effect will be realized within a relatively short time after the instillation of the cytotoxic agents, usually in about 0.5 hrs, or shortly thereafter, when inhibition of protein synthesis may be used to evaluate onset of the cytotoxic effect. However, if viability is assessed by some other mechanism, e.g., vital stains, etc., it may be hours to days before a cytotoxic effect (e.g., cell death) is noted.

The preferred method for carrying out the subject invention where the cytotoxic composition comprises complement-fixing monoclonal antibody and complement, the method involves the instillation, of about 25-200, preferably about 50-150, more preferably about 100 »l of monoclonal antibody capable of specifically binding to lens epithelial cells. Generally the monoclonal antibodies will be introduced in a physiologically acceptable solution, which may be saline, phosphate-buffered saline, or the like and will have a concentration of about 10⁸-10¹³, more usually about 10⁹-10¹² antibodies/ml. Alternatively, if performed at a time other than the initial cataract surgery, it will be necessary to either make an incision about the cornea for instillation of the monoclonal antibody, or to inject the antibody intracamerally into the anterior chamber.

After introduction of the monoclonal antibody and incubation for a sufficient time for the antibodies to bind to any remnant lens epithelial cells, usually about 30 minutes, the anterior chamber may be flushed to remove any unbound antibody. Complement (as, for example, an appropriate dilution of rabbit serum in a physiologically acceptable medium containing Mg²⁺ and Ca²⁺) is then introduced into the anterior chamber in an amount of from about 25-200, more usually from about 50-150 »l, which agent will be present in an amount sufficient to substantially completely or completely kill all of the lens epithelial cells. Generally, the cytolytic effect will be realized within a relatively short time after the introduction of complement, usually in about 0.5 hour, or shortly thereafter, when lysis of the target cells can be used to evaluate the onset of the cytolytic effect.

The subject compositions can be provided as kits for use in one or more operations. The kits will usually include the cytotoxic composition which may be present as a concentrate which may be further diluted prior to use or they may be provided at the concentration of use, where the vials may include one or more dosages. Conveniently, single dosages may be provided in syringes, contained in sterilized containers, so that the physician may employ the syringes directly, where the syringes will have the desired amount and concentration of agents. For the combination of antibody and complement, the kit may have a plurality of syringes containing the monoclonal antibodies as well as the complement in appropriate proportional amounts. Where the syringes contain the formulation for direct use, usually there will be no need for other reagents for use with the method.

The following examples are offered by way of illustration and not by way of limitation.

### EXPERIMENTAL

Murine hybridoma cell line HBI-09302, which secretes an IgM antibody substantially specific for lens epithelial cells, was deposited with the American Type Culture Collection (ATCC), 12301 Parklawn Drive, Rockville, Maryland 20852, U.S.A on March 5, 1987 and was given ATCC Accession No. HB 9343. Murine hybridoma cell line HBI-4197-X, which secretes an IgG₃ antibody substantially specific for lens epithelial cells, was deposited with the ATCC on June 14, 1988 and was given ATCC Accession No. HB 9747.

### Example 1

### Preparation of Monoclonal Antibodies

### A. Immunization

Human lens epithelial cells were obtained as small sections of the anterior capsule during cataract surgery. They were collected aseptically and placed in RPMI1640 containing 10% fetal bovine serum (FBS). Viable cells could be maintained for at least two weeks. The human lens epithelial cells were emulsified in Freunds Complete Adjuvant. Mice (BALB/c) were immunized with lens epithelial cells; 5x10⁵ cell equivalents per 200 »l were given per animal for the primary injection. Additional injections consisting of equivalent cell material emulsified in Freunds Incomplete Adjuvant were given intramuscularly 3 weeks apart. These mice received two additional injections of either ME180 cells or human amnion cells (WISH) prior to harvesting spleens for fusion.

### B. Fusion

Spleens from immunized animals were removed three to five days following the last injection and prepared as a single cell suspension. The lymphocytes then were fused with P3-X-63/Ag8.653 mouse myeloma cells under conventional conditions. Following fusion, the cells were resuspended in Iscoves medium containing hypoxanthine, aminopterin, and thymidine (HAT medium) and placed in wells according to the number of myeloma cells to give a density of about 10⁴ cells/well. The cells were fed on days 5, 12, and 15 by removing and replacing half of the medium. Cultures identified as positive for antibody secretion by screening assays, were transferred to 24 well plates containing 1 ml Iscoves medium (IMDM) containing hypoxanthine and thymidine (HT), and were cloned three times by limiting dilution.

### Example 2

### Characterization of Hybridomas 09302 and 4197-X

### Binding to Rabbit Lens Epithelial Cells

Hybridoma supernatants were initially tested for binding using rabbit lens epithelial cells. Briefly, rabbit lens epithelial cells (RLE cells) were grown in 96-well dishes and fixed with 0.025% gluteraldehyde. Culture supernatants were added and incubated for one hour at 37°C. Bound antibody was detected using goat anti-mouse IgG horse radish peroxidase. Over 800 hybrids were analyzed. One hybridoma, 09302, was selected for use in this study. The antibody binds to both human and rabbit lens epithelial cells on freshly excised anterior capsule segments. In contrast it does not bind to lymphoid cells as determined by ELISA (see Table 1).

**Table 1**

| Reactivity of Mab 09302 with Tissue Culture Cell Lines by ELISA | |
|---|---|
| Cell Lines | Reactivity* |
| Rabbit lens epithelial | +++ |
| Human lymphocyte (Daudi) | - |

| | |
|---|---|
| * ELISA absorbance value [O.D. 405 nm] | |

### Specificity of Binding

Enucleated human eyes (Lions Eye Bank, Houston, TX) were frozen in liquid nitrogen and stored at -70°C. When needed, frozen tissue sections were prepared by cryostat and mounted on glass slides. Sections were stained with hybridoma culture supernatants containing monoclonal antibodies followed by goat anti-mouse antibody conjugated to horseradish peroxidase to detect bound mouse immunoglobulin. The substrate used was 3-amino-9-ethyl carbazole. Using this technique, supernatants from the hybridoma 9302 and 4197-X bound to lens epithelial cells. Other cellular structures in the eye front were not stained, namely corneal endothelial cells, iris epithelial cells, etc. Based upon the intensity of the staining, the relative intensity for lens epithelial cells was 4+, representing 100% of the lens epithelial cells were stained; less than 10% of other cell types within the anterior chamber were stained showing that the antibodies are substantially specific for lens epithelial cells.

### Class Specificity of Monoclonal Antibody

Class and subclass of the murine hybridoma antibodies was determined using Ouchterlony analysis (see Monoclonal Antibodies: Principles and Practice, 2nd ed., Goding, Academic Press, New York, pg. 105, 1986, which publication is incorporated herein by reference). Using this technique it was determined that hybridoma 09302 produces an IgM antibody and that hybridoma 4197-X produces an IgG₃ antibody.

### Complement Fixation

A sufficient number of target cells (rabbit lens epithelial cells (RLE)) were plated in 96-well culture plates to achieve 80-90% confluence. 09302 culture supernatant or control medium (100 »l/well) was added at various dilutions to the wells and incubated for 30 minutes at 37°C. Rabbit serum (Accurate Chemical and Scientific, Westbury, NY) (5 »l/well) was added and incubation continued for 12 hrs. Living cells were identified by their ability to convert the yellow dye 3-(4,5-dimethylthiazol-2-y-l)-2,5 diphenyl tetrazolium bromide (MTT) to a purple product. Cell viability was determined based upon the intensity of color development which was proportional to viability. Intensity of colored product was measured spectrophotometrically at an optical density (O.D.) of 570 nm (Mosmann, J. Immunol. (1983) 65:55).

**Table 2**

| Effect of 09302 Antibody and Complement on RLE Cells | | | |
|---|---|---|---|
| 09302 Antibody Dilution of Culture Supernatant | | Absorbance Reading at 570 nm | |
| | | With Complement | Without Complement |
| 1 | undiluted | 0.03 | 0.24 |
| 2 | 1:3 | <0.00 | 0.33 |
| 3 | 9 | <0.00 | 0.38 |
| 4 | 27 | <0.00 | 0.38 |
| 5 | 81 | 0.04 | 0.39 |
| 6 | 243 | 0.46 | 0.41 |
| 7 | | 0.48 | 0.41 |
| Control (No antibody) | | 0.49 | 0.48 |

As shown in Table 2, the addition of complement alone had no effect on cell viability, while the combination of antibody plus complement resulted in cell destruction.

### Indirect Staining

Rabbit lens epithelial cells were seeded onto sterile coverslips. The coverslips were washed with Hanks Balanced Salt Solution (HBSS) and fixed with 3% paraformaldehyde in HBSS 24 hours prior to assay. The coverslips were again washed with HBSS and then overlaid with hybridoma supernatants. After 1 hr of incubation at room temperature, the coverslips were washed again with HBSS and overlaid with diluted fluorescein isothiocyanate-labeled goat anti-mouse immunoglobulin. The coverslips were washed, then mounted on a microscope slide. Fluorescence microscopy was done with a Zeiss microscope. Photographs were taken using Kodak Tri-X film. Results indicate that 9302 binds to surface of RLE-cells.

### Example 3

### Further Characterization of Hybridoma 4197-X

### Cell Binding of Monoclonal Antibody

The supernatant from hybridoma 4197-X was tested by ELISA for ability to bind to both normal and tumor cells. Adherent cell lines were grown to confluence in 96-well plates and then fixed with 0.05% glutaraldehyde for 10 minutes. Suspension cultures were attached to poly L-lysine coated plates and fixed as above. Monoclonal antibody in culture medium was added to the wells and incubated at 37°C for 1 hour. The plates were washed three time and bound mouse IgG detected with goat anti-mouse IgG conjugated to horse radish peroxidase. Binding of the monoclonal antibody to various cell types is shown in Table 3. The antibody is capable of binding to rabbit lens epithelial cells, as well as epithelial cell lines derived from a variety of different tissues. There is little or no binding to cells of non-epithelial origin. In addition, the antibodies from 4197-X bound to human lens epithelial cells as evaluated by immunocytochemical staining. Irrelevant antibodies did not stain human lens epithelial cells.

**Table 3**

| Binding of 4197-X Antibody to Various Cell Types | | | |
|---|---|---|---|
| Cell Line | Tissue | Cell Type | Absorbance at 450 nm* 4197-X |
| ME180 | human cervix | epithelial | 0.17 |
| WISH | human amnion | epithelial | 0.16 |
| COLO 320 | colon | adenocarcinoma | 0.00 |
| MRC5 | human skin | fibroblast | 0.06 |
| Daudi | Burkitt lymphoid | lymphoma | 0.07 |
| Y79 | retinoblastoma | | 0.06 |
| RPMI 7932 | melanoma | melanoma | 0.00 |
| RLE | rabbit lens | epithelial | 0.00 |

| | | | |
|---|---|---|---|
| * Absorbance at 450 nm: Increasing absorbance reflects increased binding of antibody to cells. | | | |

### Example 4

### Preparation of Toxin-A Chain-Antibody Conjugates

### A. Preparation Toxin A-Chain

(i) Diphtheria Toxin: Fragment A (DTA) is prepared from diphtheria toxin (Connaught Laboratories) as described (Chung and Collier, Biochem. Biophys. Acta (1977) 483:248-257) and is heated to 80°C for 10 minutes to inactivate any residual traces of toxin. Enzymic activity to DTA is assayed by ADP-ribosylation of wheat germ elongation factor 2 with ¹⁴C-NAD as substrate.
(ii) Ricin A Toxin: Ricin A chain toxin is purified from the seeds of Ricinus cummunis var zanzibariensis by a combination of affinity and ion exchange chromatographic procedures. HBI has obtained seeds through Hummert Seed Company, St. Louis, Missouri. Seeds are weighed and homogenized in 1, 1, 3 trichlorofluorethane (Freon). The defatted seed cake is then collected by vacuum filtration and is extracted with sodium acetate buffer pH 4.0. Crude extract is collected by vacuum filtration over a series of filters, the smallest being 100 micron. The filtered extract is loaded over a Fast S Sepharose cation exchange column (Pharmacia) equilibrated in the same buffer as used in the extraction. Once all of the extract is loaded, the column is washed until the effluent has returned to base line. Ricin is eluted with sodium phosphate buffer (20 mM, pH 7.5) and collected. Under these conditions, agglutinin remains bound to the column.

The Ricin fraction is loaded onto an acid treated Sepharose 4B column (Pharmacin) and the column washed until the effluent returns to base line. A reducing buffer prepared by the addition of 2-Mercapto-ethanol to 50 mM Tris pH 8.2 buffer is then passed over the column. Reduced Ricin A chain is collected. To remove contaminating B chain and whole Ricin molecules, the purified A chain is passed over a small column prepared from Fast Q Sepharose. Conditions are set such that the Ricin A does not bind to the column.

The purified Ricin A is stored in 50 mM Tris, pH 8, 1M 2-ME, concentrated to 4 mg/ml and mixed with glycerol. It is finally filter sterilized through a 0.22 micron filter into sterile vials for storage at 4°C.

### B. Synthesis of Toxin-SS Antibody Conjugates

(i) Synthesis of (DTA)-SS antibody conjugates: Antibody (7.0 ml, 2 mg/ml) is dialyzed against Dulbecco's phosphate buffered saline (DPBS Gibco), pH 7.4, containing antibiotic-antimycotic solution (Gibco, 5.0 ml/l). N-succinimidyl-3-(2-pyridyldi-thio)propionate (SPDP) (0.186 ml, 10 mM) in absolute ethanol is added to the antibody with vigorous mixing. The mixture is allowed to react for 30 min at room temperature and then dialyzed against two 1-liter changes of the same buffer. After dialysis the antibody preparation is analyzed for 2-pyridyldisulfide content as described (Stuchbury et al., Biochem. J. (1975) 151:417-432). DTA (3.0 ml, 2.5 mg/ml) is reduced by addition of 0.3 ml of 1.0 M dithiothreitol, pH 7.0, for 30 min at room temperature and desalted on Sephadex G-25 (2.6 X 12 cm column) equilibrated with the buffer described above. Peak fractions from the column are pooled (11.0 ml, 0.53 mg/ml) and mixed with PDP-(antibody) antibody (7.0 ml, 2.1 mg/ml). Final concentration of DTA and antibody are 1. 5 X 10⁻⁵M and 5.5 X 10⁻⁶M, respectively. The final molar ratio of DTA to antibody in the reaction mixture is about 3. The crude conjugate preparation (18.0 ml) is concentrated to a final volume of 0.9 ml by ultracentrifugation on an Amicon YM-10 membrane. Crude (DTA)-SS-(antibody) (9.0 ml) is chromatographed on a Sephacryl S-2000 column (2.6 X 106 cm, 22.1 ml/h flow rate) equilibrated with DPBS buffer. Each fraction (6.2 ml) is analyzed for ADP-ribosylation activity and by sodium dodecylsulfate/ polyacrylamide gel electrophoresis (SDS/PAGE). Fractions 30-40 are pooled, concentrated on an Amicon YM-10 membrane, and are used in cytotoxicity assays after filter sterilization.
(ii) Synthesis of (RTA)-SS-(antibody) antibody conjugate: (RTA)-SS(antibody) was synthesized as described by J. Biol. Chem. (1984) 133:137-146. Briefly, antibody (1 to 2 mg/ml) in 0.1 M Na bicarbonate, 0.5 M NaCl, pH 8.0, and 5-10-fold molar excess of N-succinimidyl-3-(2 pyridyldithio)propionate (SPDP) was added to the antibody with vigorous mixing. After incubation at room temperature for 30 min the pyridyldithiopropionate (PDP)-modified antibody was separated from SPDP by gel filtration over G-25 Sephodex. RTA was initially reduced by the addition of dithiothreitol at a final concentration of 50 mM, followed by incubation for 1 hour at room temperature. The RTA was then separated from DTT by gel filtration over G-25 Sephadex. The RTA was then concentrated to a final concentration of 4 mg/ml. Five- or 10-fold molar excess RTA was then added to the PDP-antibody solution and incubated for 16 hrs at 4°C. The RTA-antibody conjugate was purified by chromatography on Sephacryl S-200.

### C. Preparation of Saporin-Antibody Conjugates

(i) Isolation of Saporin
   Saporin was extracted from the seeds of Saponaria officinalis using 0.14 M NaCl, 5 mM NaPO₄, pH 7.2, using 8 ml/g, by soaking the ground seeds overnight at 4°C. The supernatant was removed and centrifuged at 28,000 X g for 30 min. The crude supernatant was dialyzed against 5 mM NaPO₄, pH 6.0, then purified by passage over a CM ion exchange column from which the saporin was eluted with an 0.0-0.3 NaCl gradient (linear). Fractions having ribosomeinhibiting activity were pooled and dialyzed against phosphate buffered saline (PBS) using the method of Stirpy et al., Virchows Arch. (1987) 53:259-271.
(ii) Conjugation
   Purified antibody is treated with N-succinimidyl-3-(2-pyridyldithio)propionate (SPDP) for 30 min at room temperature then dialyzed against PBS to remove any unreacted SPDP groups. After dialysis the antibody preparation is analyzed for 2-pyridyldisulfide content as described in Example 2.B supra. The ratio of antibody to SPDP is 10- to 15-fold excess of SPDP to antibody. The saporin is treated with a 10-fold excess of SPDP in PBS, pH 7.0, for 30 min at room temperature then desalted on Sephadex G-25 as described in Example 2.B. Peak fractions from the column are pooled and mixed with PDP-antibody in a ratio of 5-fold molar excess of saporin to antibody. The conjugates are purified by passage over a Sephacryl-300 column. Fractions having a molecular weight in the range of about 160-210 K MW are collected and tested for immunological specificity and cytotoxicity.

### Example 5

### Large Scale Antibody Production

Large scale production of a single monoclonal antibody was achieved by injecting about 10⁷ hybrid cells into appropriate H-2 compatible mice. The ascites tumors were induced by the following method. For ascites production, mice were injected intraperitoneally with 0.5 ml of pristane (2,6,10, 14-tetra-methylpentadecane, Aldrich), and rested for 1-2 months. Three to four days prior to transfer of the interspecies hybridoma, each mouse was injected with 50 » of antilymphocyte serum. On the day of tumor transfer, each mouse received total body irradiation (600-800 rads) followed 6-8 hours later by syngeneic bone marrow, 10⁷ cells mouse. Hybridoma cells (10⁶-10⁷) in Dulbeccos Modified Eagle's Medium were then injected intraperitoneally. As the tumors began to appear (10-30 days after injection), the mice were bled and the presence and concentrations of the anbtibodies in the serum continually tested. Appropriate antibodies were collected, purified and stored.

### Example 6

### Effects of Immunotoxin on Human Lens Epithelial Cells

Human lens capsules obtained from donor eyes were dissected in half along the central axis perpendicular to the equator and placed immediately on the bottom of 16 mm tissue culture wells coated with Type I collagen. One ml of medium alone (control), or containing 4197X-ricin A chain immunoconjugate (4197X-RA), were then added to the wells (day 0). Some of the samples were incubated on day 0 with whole ricin toxin (ricin AB) instead of immunoconjugate as indicated above. The wells were incubated at 37°C in 95% air-5% CO₂ until confluent cellular growth was observed on the lens explant in the control well (9-19 days). After 7 days in culture, lens explants were fed 1 ml of fresh medium (without immunotoxin). If the cells reached day 14, cultures were fed again but with 0.5 ml of medium. ³H-leucine (5 »Ci) was added to the wells in 0.1 ml of fresh medium. The samples were then incubated for 18 hours and harvested using TCA precipitation and collection on glass fiber filters for the determination of ³H-leucine incorporation into protein.

**Table 4**

| Effects of Continuous Exposure to Immunotoxin on Protein Synthesis in Human Lens Epithelial Cells | | | | |
|---|---|---|---|---|
| Sample | Treatment | | ³H-leucine Incorporation | |
| | | | dpm* | % Inhibition |
| 89-1171 | control | | 612.1 | |
| 89-1171 | ricin AB | 20 »g/ml | 38.1 | 93.8 |
| 89-1154 | control | | 1106.6 | |
| 89-1154 | 4197X-RA | 50 »g/ml | 46.8 | 95.8 |
| 89-132 | control | | 15046.9 | |
| 89-132 | 4197X-RA | 5 »g/ml | 27.5 | 99.7 |
| 89-1194 | control | | 2371.8 | |
| 89-1194 | 4197X-RA | 5 »g/ml | 69.3 | 97.1 |
| 89-1220 | control | 0.05 »g/ml | 11.6 | 99.8 |

| | | | | |
|---|---|---|---|---|
| *dpm given represents the TCA-precipitable radioactivity incorporated into the lens tissue over 18 hours minus the 0 time background binding of ³H-leucine to tissue specimens alone (37.7 dpm). | | | | |

By introducing the subject cytotoxic agents, particularly after introduction of the non-cytotoxic binding moiety, remnant lens epithelial cells can be prevented from proliferating, thus avoiding secondary cataracts. The subject methods and compositions provide a simple procedure for preventing secondary cataracts while avoiding injury to other tissues in the eye and provide a safe alternative to the various techniques which have been used previously but which have general cytotoxic effects. By the subject two-stage treatment, a relatively non-specific cytotoxic agent (for example an antibody specific for epithelial cells) can be made specific for a certain epithelial cell population, lens epithelial cells. The unique anatomical location of the various epithelial cell types during cataract surgery renders the treatment method possible.

All publications and patent applications mentioned in this specification are indicative of the level of skill of those skilled in the art to which this invention pertains. All publications and patent applications are herein incorporated by reference to the same extent as if each individual publication or patent application was specifically and individually indicated to be incorporated by reference.

The invention now being fully described, it will be apparent to one of ordinary skill in the art that many changes and modifications can be made thereto without departing from the spirit or scope of the appended claims.

## Claims

1. A composition comprising:
a hybridoma cell culture producing antibodies which bind substantially specifically to human lens epithelial cells, said hybridoma cell culture having ATCC Accession No. HB9343 or HB9747.

2. A composition comprising:
a monoclonal antibody which binds substantially specifically to an antigenic determinant on the surface of a human lens epithelial cell, and wherein said antibody is classified as an IgM, IgG, 2a, 2b, or 3 antibody, or the human equivalent thereof, wherein said antibody is produced by a hybridoma cell culture having ATCC Accession No. HB9343 or HB9747.

3. A cytotoxic composition comprising:
a monoclonal antibody which binds substantially specifically with normal human lens epithelial cells, wherein said antibodies are produced by hybridoma cells having ATCC Accession No. HB 9343 or RB9747.

4. A cytotoxic composition according to Claim 3, wherein said monoclonal antibody is a complement-activating antibody.

5. A cytotoxic composition according to Claim 3, wherein said monoclonal antibody is conjugated to a toxic agent.

6. A cytotoxic composition according to Claim 5, wherein said toxic agent is the A chain of diphtheria toxin; ricin; or abrin.

7. A cytotoxic composition according to Claim 5, wherein said toxic agent is saporin.

8. A kit comprising a first container containing a complement-fixing monoclonal antibody substantially specific for human lens epithelial cells and produced by hybridoma cells having ATCC Accession No. HB9343 or HB9747; and a second container containing complement.

9. A kit according to Claim 8, wherein said first and second containers comprise syringes.

10. A kit comprising a first container containing a cytotoxic agent substantially specific for human lens epithelial cells and a second container containing a non-cytotoxic agent cross-reactive with said cytotoxic agent, wherein said cytotoxic agent comprises a monoclonal antibody produced by hybridoma cells having ATCC Accession No. HB9343 or RB9747.

## Patentansprüche

1. Zusammensetzung, umfassend:
eine Hybridomzellkultur, die Antikörper produziert, welche im wesentlichen spezifisch an Epithelzellen der menschliche Linse bindet, wobei die Hybridomzellkultur die ATCC-Zugangsnummer HB9343 oder HB9747 besitzt.

2. Zusammensetzung, umfassend:
einen monoklonalen Antikörper, welcher im wesentlichen spezifisch an eine antigene Determinante auf der Oberfläche einer Epithelzelle der menschlichen Linse bindet, und wobei der Antikörper als ein IgM-, IgG-, 2a-, 2b- oder 3-Antikörper klassifiziert ist, oder das menschliche Äquivalent davon, wobei der Antikörper von einer Hybridomzellkultur der ATCC-Zugangsnummer HB9343 oder HB9747 produziert wird.

3. Cytotoxische Zusammensetzung, umfassend:
einen monoklonalen Antikörper, der im wesentlichen spezifisch an normalen Epithelzellen der menschlichen Linse bindet, wobei die Antikörper von Hybridomzellen der ATCC-Zugangsnummer HB 9343 oder HB 9747 produziert werden.

4. Cytotoxische Zusammensetzung gemäß Anspruch 3, worin der monoklonale Antikörper ein Komplement-aktivierender Antikörper ist.

5. Cytotoxische Zusammensetzung gemaß Anspruch 3, worin der monoklonale Antikörper mit einer toxischen Substanz konjugiert ist.

6. Cytotoxische Zusammensetzung gemäß Anspruch 5, worin die toxische Substanz die A-Kette des Diphtherie-Toxis; Ricin; oder Abrin ist.

7. Cytotoxische Zusammensetzung gemäß Anspruch 5, worin die toxische Substanz Saporin ist.

8. Kit, umfassend einen ersten Behälter, der einen Komplement-fixierenden monoklonaleen Antikörper, welcher im wesentlichen für Epithelzellen der menschlichen Linse spezitisch ist und Von Hybridomzellen der ATCC-Zugangsnummer HB9343 oder HB9747 produziert wird, enthält; und einen zweiten, Komplement enthaltenden Behälter.

9. Kit gemäß Anspruch 8, worin der erste und zweite Behälter Spritzen umfassen.

10. Kit, umfassend einen ersten Behälter, der eine im wesentlichen für Epithelzellen der menschlichen Linse spezifische cytotoxische Substanz enthält, und einen zweiten Behälter, der eine mit der cytotoxischen Substanz kreuzreaktive nicht-cytotoxische Substanz enthält, worin die cytotoxische Substanz einen von Hybridomzellen der ATCC-Zugangsnummer HB9343 oder HB9747 produzierten monoklonalen Antikörper umfaßt.

## Revendications

1. **Composition comprenant :**
une culture cellulaire d'hybridomes produisant des anticorps qui se lient de manière sensiblement spécifique aux cellules épithéliales du cristallin humain, ladite culture cellulaire d'hybridomes ayant le numéro de dépôt ATCC n° HB9343 ou HB9747.

2. **Composition comprenant :**
un anticorps monoclonal qui se lie de manière sensiblement spécifique à un déterminant antigénique à la surface d'une cellule épithéliale du cristallin humain, et dans laquelle ledit anticorps est classé comme anticorps IgM, IgG, 2a, 2b, ou 3, ou l'équivalent humain de celui-ci, où ledit anticorps est produit par une culture cellulaire d'hybridomes ayant le numéro de dépôt ATCC n⁰ HB9343 ou HB9747.

3. **Composition cytotoxique comprenant :**
un anticorps monoclonal qui se lie de manière sensiblement spécifique aux cellules épithéliales normales du cristallin humain, dans laquelle lesdits anticorps sont produits par des cellules d'hybridomes ayant le numéro de dépôt ATCC n° HB9343 ou HB9747.

4. Composition cytotoxique selon la revendication 3, dans laquelle ledit anticorps monoclonal est un anticorps activateur du complément.

5. Composition cytotoxique selon la revendication 3, dans laquelle ledit anticorps monoclonal est conjugué à un agent toxique.

6. Composition cytotoxique selon la revendication 5, dans laquelle ledit agent toxique est la chaîne A de la toxine de la diphtérie ; la ricine ; ou l'abrine.

7. Composition cytotoxique selon la revendication 5, dans laquelle ledit agent toxique est la saporine.

8. Nécessaire comprenant un premier récipient contenant un anticorps monoclonal fixant le complément sensiblement spécifique des cellules épithéliales du cristallin humain et produit par des cellules d'hybridomes ayant le numéro de dépôt ATCC n° HB9343 ou HB9747 ; et un second récipient contenant du complément.

9. Nécessaire selon la revendication 8, dans lequel lesdits premier et second récipients comprennent des seringues.

10. Nécessaire comprenant un premier récipient contenant un agent cytotoxique sensiblement spécifique des cellules épithéliales du cristallin humain et un second récipient contenant un agent non cytotoxique produisant une réaction croisée avec ledit agent cytotoxique, dans lequel ledit agent cytotoxique comprend un anticorps monoclonal produit par des cellules d'hybridomes ayant le numéro de dépôt ATCC n° HB9343 ou HB9747.
